(19) **Europäisches Patentamt**

**European Patent Office**

**Office européen des brevets**

(11) **EP 0 898 954 B1**

(12) **EUROPÄISCHE PATENTSCHRIFT**

(45) Veröffentlichungstag und Bekanntmachung des Hinweises auf die Patenterteilung:
**15.09.2004 Patentblatt 2004/38**

(51) Int Cl.[7]: **A61K 7/13**, D06P 1/34, D06P 1/19

(21) Anmeldenummer: **98111107.3**

(22) Anmeldetag: **17.06.1998**

(54) **Mittel zum Färben von Keratinfasern**

Composition for dyeing keratin fibres

Composition de teinture des fibres kératiniques

(84) Benannte Vertragsstaaten:
**DE ES FR GB IT**

(30) Priorität: **22.08.1997 DE 19736553**

(43) Veröffentlichungstag der Anmeldung:
**03.03.1999 Patentblatt 1999/09**

(73) Patentinhaber: **Wella Aktiengesellschaft**
**64274 Darmstadt (DE)**

(72) Erfinder:
• **Sallwey, Anette**
**63303 Dreieich (DE)**

• **Schmitt, Manfred**
**64646 Heppenheim (DE)**
• **Lenz, Uwe, Dr.**
**64673 Zwingenberg (DE)**

(56) Entgegenhaltungen:
**EP-A- 0 782 845       DE-A- 4 233 874**

Bemerkungen:
Die Akte enthält technische Angaben, die nach dem Eingang der Anmeldung eingereicht wurden und die nicht in dieser Patentschrift enthalten sind.

Anmerkung: Innerhalb von neun Monaten nach der Bekanntmachung des Hinweises auf die Erteilung des europäischen Patents kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist. (Art. 99(1) Europäisches Patentübereinkommen).

**Beschreibung**

Gegenstand der vorliegenden Erfindung ist ein Mittel zum Färben von Keratinfasern, insbesondere menschlichen Haaren, auf der Basis von getrockneten und gemahlenen Indigoblättern und bestimmten synthetischen direktziehenden Farbstoffen.

Die Nuancierungsmöglichkeiten von Färbemitteln, welche nur auf natürlichen Bestandteilen basieren, sind äußerst begrenzt. Dies liegt zum einen an der geringen Auswahl der zu Verfügung stehenden Naturfarbstoffe und zum anderen an dem spezifischen Verhalten dieser Naturstoffe. So ergeben gemahlene Indigoblätter unmittelbar nach der Färbung ein grün-blaues Farbbild, das innerhalb von 72 Stunden nach der Behandlung in ein blau-rotes Farbbild umschlägt. Ebenso ist mit solchen Haarfärbemitteln nur eine unzureichende Abdeckung grauer Haare zu erzielen. Zur Vermeidung von ungewollten färberischen Effekten wird zudem bei vielen der sogenannten natürlichen Fäbemittel" oder Färbemitteln auf pflanzlicher Basis" auf färbende pflanzliche Bestandteile verzichtet und stattdessen auf eine Kombination aus synthetischen Farbstoffen und nichtfärbenden natürlichen Bestandteilen zurückgegriffen.

Insbesondere blaue und blaurote Färbungen sind mit natürlichen Farbstoffen, aber auch mit direktziehenden Farbstoffen, nur schwer zu erzielen.

Es bestand daher die Aufgabe, ein Färbemittel auf der Basis von Indigo zur Verfügung zu stellen, welches die vorstehend beschriebenen Nachteile nicht aufweist, insbesondere blaue Nuancierungen mit natürlichen Pflanzenbestandteilen beziehungsweise Pflanzenfarben ermöglicht und dessen Farbbild sich nach der Applikation im Vergleich zum Stand der Technik nur noch wenig ändert.

Überraschender Weise wurde nunmehr gefunden, daß bei Verwendung einer Kombination aus Indigo und bestimmten direktziehenden Farbstoffen, der bei Färbungen mit Indigoblättern üblicherweise auftretende Farbumschlag von blau-grün nach blau-rot vermieden werden kann. Weiterhin wurde überraschender Weise gefunden, daß bei pflanzlichen Bestandteile beziehungsweise Pflanzenfarben enthaltenden Färbemitteln durch die Verwendung einer Kombination aus Indigo und bestimmten synthetischen direktziehenden Farbstoffen eine Verstärkung der Farbleistung und der Deckkraft gegenüber Mitteln, welche ausschließlich synthetische Farbstoffe ohne Indigozusatz enthalten, erzielt wird.

Gegenstand der vorliegenden Erfindung ist daher ein Mittel zum Färben von Keratinfasern, insbesondere menschlichen Haaren, welches dadurch gekennzeichnet ist, daß es eine Kombination aus (a) getrockneten und gemahlenen Indigoblättern und (b) mindestens einem synthetischen direktziehenden Farbstoff, welcher ausgewählt ist aus der Gruppe bestehend aus 4-[Ethyl-(2-hydroxyethyl)amino]-1-[(2-hydroxyethyl)amino]-2-nitrobenzol (Hydrochlorid: HC Blue No. 12), 1-(2,3-Dihydroxypropyl)-amino-2-nitro-4-[methyl-(2-hydroxyethyl)amino]-benzol (Hydrochlorid: HC Blue No. 10), 1-(2,3-Dihydroxypropyl)amino-2-nitro-4-[ethyl-(2-hydroxyethyl)amino]-benzol (Hydrochlorid: HC Blue No. 9), 1-(3-Hydroxypropyl)-amino-2-nitro-4-bis-(2-hydroxyethylamino)-benzol (HC Violet No. 2), 1-(2-Hydroxyethyl)amino-2-nitro-4-amino-benzol (HC Red No. 3) und 1-Amino-4-[di(2-hydroxyethyl)amino]-2-nitrobenzol (Hydrochlorid: HC Red No. 13), enthält, wobei die vorgenannten synthetischen direktziehenden Farbstoffe sowohl in Form der freien Base als auch in Form der physiologisch verträglichen Salze vorliegen können.

Besonders bevorzugt ist hierbei eine Kombination aus (a) getrockneten und gemahlenen Indigoblättern und (b) mindestens einem synthetischen direktziehenden Farbstoff welcher ausgewählt ist aus der Gruppe bestehend aus 4-[Ethyl-(2-hydroxyethyl)amino]-1-[(2-hydroxyethyl)amino]-2-nitrobenzol (Hydrochlorid: HC Blue No. 12) und 1-Amino-4-[di(2-hydroxyethyl)amino]-2-nitrobenzol (Hydrochlorid: HC Red No. 13) oder deren Salzen, enthält.

Die Gesamtmenge, der vorstehend genannten synthetischen direktziehenden Farbstoffe in dem Färbemittel beträgt 0,05 bis 10 Gewichtsprozent, insbesondere 0,1 bis 8 Gewichtsprozent, wobei eine Einsatzmenge von 0,1 bis 5 Gewichtsprozent besonders bevorzugt ist. Die Einsatzmenge der vorstehend genannten synthetischen direktziehenden Farbstoffe in dem (gegebenenfalls mit Wasser vermischten) gebrauchsfertigen Färbemittelgemisch, beträgt vorzugsweise 0,01 bis 3,5 Gewichtsprozent.

Die Einsatzmenge der getrockneten und gemahlenen Indigoblätter in dem Färbemittel beträgt 0,5 bis 99,95 Gewichtsprozent, insbesondere 1 bis 90 Gewichtsprozent. Die Einsatzmenge der getrockneten und gemahlenen Indigoblätter, bezogen auf das (gegebenenfalls mit Wasser vermischte) gebrauchsfertige Färbemittel, beträgt 0,1 bis 35 Gewichtsprozent.

Das erfindungsgemäße Färbemittel kann zusätzlich weitere färbende pflanzliche Bestandteile, wie zum Beispiel Hennablätter, Kamillenblüten, Curcuma-Wurzeln, Faulbaumrinde, Olivenblätter, kanadische Blutwurzel,Gelbwurzel, Gelbholz, Rotholz, Rotsandelholz, Blauholz, Krappwurzel, Schwarzer Holunder oder Schwarze Apfelbeere oder deren Kombinationen enthalten, wobei die Gesamtmenge dieser Stoffe bezogen auf die Gesamtmenge des Färbemittelgemisches 3 bis 95 Gewichtsprozent, insbesondere von 5 bis 90 Gewichtsprozent, betragen kann.

Ebenfalls können dem erfindungsgemäßen Färbemittel zur Erzielung bestimmter Farbnuancen übliche, physiologisch unbedenkliche, direktziehende Farbstoffe aus der Gruppe der Nitrofarbstoffe, Azofarbstoffe, Chinonfarbstoffe und Triphenylmethanfarbstoffe wie zum Beispiel 1,4-Bis[(2-hydroxyethyl)amino]-2-nitrobenzol, 1-Amino-3-methyl-4-[(2-hydroxyethyl)amino]-6-nitrobenzol (HC Violet No. 1), 4-[Di(2-hydroxyethyl)amino]-1-[(2-methoxyethyl)amino]-2-nitrobenzol (HC Blue No. 11), 1-Methylamino-4-[methyl-(2,3-dihydroxypropyl)amino]-2-nitrobenzol (HC Blue No. 6), 2-((4-Amino-2-nitrophenyl)amino)-5-dimethylamino-benzoesäure (HC Blue No. 13), 1-Amino-4-[(2-hydroxyethyl) amino]-2-nitrobenzol (HC Red No. 7), 2-Amino-4,6-dinitrophenol, 4-Amino-2-nitro-diphenylamin (HC Red No. 1), 1-Amino-5-chlor-4-[(2-hydroxyethyl)amino]-2-nitrobenzol, 4-Amino-3-nitrophenol, 4-[(2-Hydroxyethyl)amino]-3-nitrophenol, 1-[(2-Aminoethyl)amino]-4-(2-hydroxyethoxy)-2-nitrobenzol (HC Orange No. 2), 4-(2,3-Dihydroxypropoxy)-1-[(2-hydroxyethyl)amino]-2-nitrobenzol (HC Orange No. 3), 1-Amino-5-chlor-4-[(2,3-dihydroxypropyl)amino]-2-nitrobenzol (HC Red No. 10), 5-Chlor-1,4-[di(2,3-dihydroxypropyl)amino] -2-nitrobenzol (HC Red No. 11), 2-[(2-Hydroxyethyl)amino]-4,6-dinitro-phenol, 4-Ethylamino-3-nitrobenzoesäure, 2-[(4-Amino-2-nitrophenyl)amino]-benzoesäure, 2-Chlor-6-ethylamino-4-nitrophenol, 2-Amino-6-chlor-4-nitrophenol, 4-[(3-Hydroxypropyl)amino]-3-nitrophenol, 2,5-Diamino-6-nitropyridin, 1,2,3,4-Tetrahydro-6-nitrochinoxalin, 7-Amino-3,4-dihydro-6-nitro-2H-1,4-benzoxazin (HC Red No. 14), 1-Amino-2-[(2-hydroxyethyl)amino]-5-nitrobenzol (HC Yellow No. 5), 1-(2-Hydroxyethoxy)-2-[(2-hydroxyethyl) amino]-5-nitrobenzol (HC Yellow No. 4), 1-[(2-Hydroxyethyl)amino]-2-nitrobenzol (HC Yellow No. 2), 2-[(2-Hydroxyethyl)amino]-1-methoxy-5-nitrobenzol, 2-Amino-3-nitrophenol, 1-(2-Hydroxyethoxy)-3-methylamino-4-nitrobenzol, 2,3-(Dihydroxypropoxy)-3-methylamino-4-nitrobenzol, 2-[(2-Hydroxyethyl)amino]-5-nitrophenol (HC Yellow No. 11), 3-[(2-Aminoethyl)amino]-1-methoxy-4-nitrobenzol-hydrochlorid (HC Yellow No.9), 1-[(2-Ureidoethyl)amino]-4-nitrobenzol, 4-[(2,3-Dihydroxypropyl)amino]-3-nitro-1-trifluormethyl-benzol (HC Yellow No. 6), 1-Chlor-2,4-bis[(2-hydroxyethyl)amino]-5-nitrobenzol (HC Yellow No. 10), 4-[(2-Hydroxyethyl)amino]-3-nitro-1-methylbenzol, 1-Chlor-4-[(2-hydroxyethyl)amino]-3-nitrobenzol (HC Yellow No. 12), 4-[(2-Hydroxyethyl)amino]-3-nitro-1-trifluormethyl-benzol (HC Yellow No. 13), 4-[(2-Hydroxyethyl)amino]-3-nitro-benzonitril (HC Yellow No. 14), 4-[(2-Hydroxyethyl)amino]-3-nitro-benzamid (HC Yellow No. 15), 1,4-Di[(2,3-dihydroxypropyl)amino]-9,10-anthrachinon, 1-[(2-Hydroxyethyl)amino]-4-methylamino-9,10-anthrachinon (CI61505, Disperse Blue No. 3), 2-[(2-Aminoethyl)amino]-9,10-anthrachinon (HC Orange No. 5), 1-Hydroxy-4-[(4-methyl-2-sulfophenyl)amino]-9,10-anthrachinon, 1-[(3-Aminopropyl)amino]-4-methylamino-9,10-anthrachinon (HC Blue No. 8), 1-[(3-Aminopropyl)amino]-9,10-anthrachinon (HC Red No. 8), 1,4-Diamino-2-methoxy-9,10-anthrachinon (CI62015, Disperse Red No. 11, Solvent Violet No. 26), 1,4-Dihydroxy-5,8-bis[(2-hydroxyethyl)amino]-9,10-anthrachinon (CI62500, Disperse Blue No. 7, Solvent Blue No. 69), 9-(Dimethylamino)-benzo[a]phenoxazin-7-iumchlorid (CI51175; Basic Blue No. 6), Di[4-(diethylamino)phenyl][4-(ethylamino)naphthyl]carbeniumchlorid (CI42595; Basic Blue No. 7), 3,7-Di(dimethylamino)phenothiazin-5-ium-chlorid (CI52015; Basic Blue No. 9), Di[4-(dimethylamino)phenyl][4-(phenylamino)naphthyl]-carbeniumchlorid (CI44045; Basic Blue No. 26), 2-[(4-(Ethyl(2-hydroxyethyl)amino)-phenyl)azo]-6-methoxy-3-methyl-benzothiazolium-methylsulfat (CI11154; Basic Blue No. 41), 8-Amino-2-brom-5-hydroxy-4-imino-6-[(3-(trimethylammonio)phenyl)amino]-1(4H)-naphthalinon-chlorid (CI56059; Basic Blue No. 99), Bis[4-(dimethylamino)phenyl][4-(methylamino)phenyl]carbeniumchlorid (CI42535; Basic Violet No. 1), Tris[4-(dimethylamino)phenyl]-carbenium-chlorid (CI42555; Basic Violet No. 3), 2-[3,6-(Diethylamino)-dibenzopyranium-9-yl]-benzoesäure-chlorid (CI45170; Basic Violet No. 10), Di(4-aminophenyl)(4-amino-3-methylphenyl)carbenium-chlorid (CI42510; Basic Violet No. 14), 1,3-Bis[(2,4-diamino-5-methylphenyl)azo]-3-methylbenzol (CI21010;Basic Brown No. 4), 1-[(4-Aminophenyl)azo]-7-(trimethylammonio)-2-naphthol-chlorid (CI12250; Basic Brown No. 16), 1-[(4-Amino-2-nitrophenyl)azo]-7-(trimethylammonio)-2-naphthol-chlorid (Basic Brown No. 17), 1-[(4-Amino-3-nitrophenyl)azo]-7-(trimethylammonio)-2-naphthol-chlorid (CI12251; Basic Brown No. 17), 3,7-Diamino-2,8-dimethyl-5-phenylphenazinium-chlorid (CI50240; Basic Red No. 2), 1,4-Dimethyl-5-[(4-(dimethylamino)phenyl)-azo]-1,2,4-triazolium-chlorid (CI11055; Basic Red No. 22), 2-Hydroxy-1-[(2-methoxyphenyl)azo]-7-(trimethylammonio)-naphthalin-chlorid (CI12245; Basic Red No. 76), 2-[2-((2,4-Dimethoxyphenyl)amino)ethenyl]-1,3,3-trimethyl-3H-indol-1-ium-chlorid (CI48055; Basic Yellow No. 11), 3-Methyl-1-phenyl-4-[(3-(trimethylammonio)phenyl)azo]-pyrazol-5-on-chlorid (CI12719; Basic Yellow No. 57), Bis[4-(diethylamino)phenyl]-phenylcarbenium-hydrogensulfat(1:1) (CI42040; Basic Green No. 1), 1-[Di(2-hydroxyethyl)amino]-3-methyl-4-[(4-nitrophenyl)azo]-benzol (CI11210, Disperse Red No. 17), 4-[(4-Aminophenyl)azo]-1-[di(2-hydroxyethyl)amino]-3-methylbenzol (HC Yellow No. 7), 2,6-Diamino-3-[(pyridin-3-yl)azo]-pyridin, 6-Hydroxy-5-[(4-sulfophenyl)azo]-2-naphthalinsulfonsäure-dinatriumsalz (CI15985), 2,4-Dinitro-1-naphthol-7-sulfonsäure-dinatriumsalz (CI10316; Acid Yellow No. 1), 2-(Indan-1,3-dion-2-yl)chinolin-x,x-sulfonsäure (Gemisch aus Mono- und Disulfonsäure) (CI47005; Acid Yellow No. 3), 5-Hydroxy-1-(4-sulfophenyl)-4-[(4-sulfophenyl)azo]pyrazol-3-carbonsäuretrinatriumsalz (CI19140; Acid Yellow No. 23), 9-(2-Carboxyphenyl)-6-hydroxy-3H-xanthen-3-on (CI45350; Acid Yellow No. 73), 5-[(2,4-Dinitrophenyl)amino]-2-phenylamino-benzolsulfonsäurenatriumsalz (CI10385; Acid Orange No. 3), 4-[(2,4-Dihydroxyphenyl)azo]-benzolsulfonsäure-mononatriumsalz (CI14270; Acid Orange No. 6), 4-[(2-Hydroxynaphth-1-yl)azo]-benzolsulfonsäure-natriumsalz (CI15510; Acid Orange No. 7), 4-[(2,4-Dihydroxy-3-[(2,4-dimethylphenyl)azo]phenyl)azo]-benzolsulfonsäurenatriumsalz (CI20170; Acid Orange No. 24), 4-Hydroxy-3-[(4-sulfonaphth-1-yl)azo]-1-naphthalin-sulfonsäure-dinatriumsalz (CI14720; Acid Red No. 14), 6-Hydroxy-5-[(4-sulfonaphth-1-yl)azo]-2,4-naphthalin-disulfonsäuretrinatriumsalz (CI16255; Acid Red No. 18), 3-Hydroxy-4-[(4-sulfonaphth-1-yl)azo]-2,7-naphthalin-disulfonsäure-trinatriumsalz (CI16185; Acid Red No. 27), 8-Amino-1-hydroxy-2-(phenylazo)-3,6-naphthalin-disulfonsäuredinatriumsalz (CI17200; Acid Red No. 33), 5-(Acetylamino)-4-hydroxy-3-[(2-methylphenyl)azo]-2,7-naphthalindisulfonsäure-dinatriumsalz (CI18065; Acid Red No. 35), 2-(3-Hydroxy-2,4,5,7-tetraiod-dibenzopyran-6-on-9-yl)-benzoesäure-dinatriumsalz (CI45430;Acid Red No. 51), N-[6-(Diethylamino)-9-(2,4-disulfophenyl)-3Hxanthen-3-yliden]-N-ethylethanammonium-hydroxid, inneres Salz, Natriumsalz (CI45100; Acid Red No. 52), 8-[(4-(Phenylazo)phenyl)azo]-7-naphthol-1,3-disulfonsäure-dinatriumsalz (CI27290; Acid Red No. 73), 2',4',5',7'-Tetrabrom-3',6'-dihydroxyspiro-[isobenzofuran-1(3H),9'-[9H]xanthen]-3-on-dinatriumsalz (CI45380; Acid Red No. 87), 2', 4',5',7'-Tetrabrom-4,5,6,7-tetrachlor-3',6'-dihydroxyspiro[isobenzofuran-1(3H),9'[9H]xanthen]-3-on-dinatriumsalz (CI45410; Acid Red No. 92), 3',6'-Dihydroxy-4',5'-diiodospiro[isobenzofuran-1(3H),9'(9H)-xanthen]-3-on-dinatriumsalz (CI45425; Acid Red No. 95), (2-Sulfophenyl)di[4-(ethyl((4-sulfophenyl)methyl)amino)phenyl]-carbenium-dinatriumsalz, betain (CI42090; Acid Blue No. 9), 1,4-Bis[(2-sulfo-4-methylphenyl)amino]-9,10-anthrachinon-dinatriumsalz (CI 61570; Acid Green No. 25), Bis[4-(dimethylamino)phenyl]-(3,7-disulfo-2-hydroxynaphth-1-yl)carbenium-inneres Salz, mononatriumsalz (CI44090; Acid Green No. 50), Bis[4-(diethylamino)phenyl](2,4-disulfophenyl) carbenium-inneres salz, Natriumsalz (2:1) (CI42045; Acid Blue No. 1), Bis[4-(diethylamino)phenyl](5-hydroxy-2,4-disulfophenyl)carbeniuminneres salz, Calciumsalz (2:1) (CI42051; Acid Blue No. 3), 1-Amino-4-(cyclohexylamino)-9,10-anthrachinon-2-sulfonsäure-natriumsalz (CI62045; Acid Blue No. 62), 2-(1,3-Dihydro-3-oxo-5-sulfo-2H-indol-2-yliden)-2,3-dihydro-3-oxo-1H-indol-5-sulfonsäure-dinatriumsalz (CI73015; Acid Blue No. 74), 9-(2-Carboxyphenyl)-3-[(2-methylphenyl)amino]-6-[(2-methyl-4-sulfophenyl)-amino]xanthylium-inneres Salz, mononatriumsalz (CI45190; Acid Violet No. 9), 1-Hydroxy-4-[(4-methyl-2-sulfophenyl)amino]-9,10-anthrachinon-natriumsalz (CI60730; Acid Violet No. 43), Bis[3-nitro-4-[(4-phenylamino)-3-sulfo-phenylamino]-phenyl]-sulfon (CI10410; Acid Brown No. 13), 5-Amino-4-hydroxy-6-[(4-nitrophenyl)azo]-3-(phenylazo)-2,7-naphthalin-disulfonsäure-dinatriumsalz (CI20470; Acid Black No. 1), 3-Hydroxy-4-[(2-hydroxynaphth-1-yl)azo]-7-nitro-1-naphthalin-sulfonsäure-chromkomplex (3:2) (CI15711; Acid Black No. 52), 3-[(2,4-Dimethyl-5-sulfophenyl)azo]-4-hydroxy-1-naphthalinsulfonsäure-dinatriumsalz (CI14700), 4-(Acetylamino)-5-hydroxy-6-[(7-sulfo-4-[(4-sulfophenyl)azo]naphth-1-yl)azo]-1,7-naphthalindisulfonsäuretetranatriumsalz (CI28440) und 3-Hydroxy-4-(3-methyl-5-oxo-1-phenyl-4,5-dihydro-1H-pyrazol-4-ylazo)-naphthalin-1-sulfonsäure-natriumsalz, Chrom-Komplex (Acid Red No. 195), alleine oder in Kombination miteinander, zugesetzt werden, wobei diese Farbstoffe in einer Gesamtmenge von, bezogen auf die Gesamtmenge des Färbemittelgemisches, 0,01 bis 15 Gewichts-prozent, insbesondere 0,1 bis 12 Gewichtsprozent, zugesetzt werden.

Zusätzlich kann das erfindungsgemäße Färbemittel weitere für derartige Zusammensetzungen übliche Zusatzstoffe, wie zum Beispiel Parfümöle, färbende tierische Bestandteile wie Cochenille oder Lac Dye, nicht-färbende pflanzliche Bestandteile wie beispielsweise Cassia Auriculata (Henna, neutral), Fichtennadeln, Akazienblätter, Lindenblätter, Birkenblätter, Weizen, Roggen, Gerste, Süßholz, Catechu oder Salbeikraut oder deren Kombinationen, sowie Verdicker wie Cellulosen, Alginate, Polysaccharide oder mineralische Verdicker wie Bentonit, Tenside und Emulgatoren, Antioxidantien wie Butylhydroxyanisol, Butyhydroxytoluol oder Tocopherol, Fruchtwachse, Chelatisierungsmittel oder Lösungsvermittler wie Polyethylenglykole, beispielsweise Polyethylenglykol(4), enthalten.

Weiterhin kann das erfindungsgemäße Färbemittel zur Einstellung des gewünschten pH-Wertes Akalisierungsmittel, wie zum Beispiel Alkalihydroxide, Natriumcarbonat, Natriumhydrogencarbonat, Magnesiumcarbonat, Ammoniumcarbonat, Ammoniumhydrogencarbonat oder Natriumsilikate, oder Ansäuerungsmittel, wie zum Beispiel Zitronensäure, Weinsäure, Ammoniumchlorid oder Ammoniumsulfat, enthalten.

Das erfindungsgemäße Färbemittel kann sowohl in Form eines Pulvers, eines Granulates, einer Emulsion, einer Dispersion oder einer Supension vorliegen.

Das erfindungsgemäße Färbemittel kann weiterhin sowohl in Form einer Einkomponenten-Zubereitung als auch in Form einer Mehrkomponenten-Zubereitung zur Anwendung kommen, wobei im Falle der Mehrkomponenten-Zubereitung die natürlichen Bestandteile getrennt von den übrigen Bestandteilen abgepackt werden und unmittelbar vor der Anwendung beziehungsweise vor dem Vermischen mit Wasser zusammengegeben werden.

Als Öle oder Wachse für die genannten emulsionsförmigen, dispersionsförmigen oder supensionsförmigen Färbemittel eignen sich insbesondere flüssige Silikone oder Paraffine, pflanzliche oder tierische Öle, flüssige Fettsäuren, flüssige Fettsäureester, flüssige alkoxylierte Fettsäureester, flüssige Glyceride, flüssige alkoxylierte Glyceride, flüssige Fettalkohole, flüssige alkoxylierte Fettalkohole, insbesondere flüssige Fettalkoholethoxylate mit 2 bis 5 Ethylenoxideinheiten im Molekül, flüssige mehrwertige Alkohole, flüssige Nonylphenolether und flüssige Polypropylenglykole oder wachsförmige Silikone oder Paraffine, pflanzliche oder tierische Wachse, Fettsäuren, Fettsäureester, Glyceride, Monodiglyceride, Fettalkohole, alkoxylierte Fettalkohole, insbesondere, Polyethylenglykole oder Polypropylenglykole, sowie Glyceride.

Die Öle und Wachse können, bezogen auf das gebrauchsfertige Färbemittel, in einer Gesamtmenge von 0,1 bis 35 Gewichtsprozent eingesetzt werden.

Sofern erforderlich, kann das erfindungsgemäße Färbemittel zusätzlich Tenside und Emulgatoren aus der Gruppe der nichtionischen, kationischen, anionischen oder amphoteren oberflächenaktiven Verbindungen beziehungsweise O/W- oder W/O-Emulgatoren, wie zum Beispiel Silikontenside, Alkyletherphosphate, Glyceridalkoxylate, Alkylsulfate, Alkalimetallsalze oder Erdalkalimetallsalze von Fettsäuren, Alkylethersulfate, Alkylsulfoacetate, oxethylierte Fettsäureester und Fettalkoholalkoxylate, enthalten.

Vorzugsweise wird das Färbemittelgemisch vor der Anwendung mit kaltem oder warmen (T = 10 bis 100 °C) Wasser zu einem auftragefähigen Färbebrei vermischt, wobei dieses Vermischen in einer Schale oder durch Anschütteln in einer Auftrageflasche erfolgen kann.

Das Mischungsverhältnis von Färbemittel zu Wasser beträgt vorzugsweise etwa 1 : 1 bis 1 : 100, wobei ein Mischungsverhältnis von 1 : 2 bis 1 : 50 besonders bevorzugt ist.

Im Falle der Mehrkomponenten-Zubereitung werden die natürlichen Bestandteile mit den übrigen Bestandteilen vor der Anwendung beziehungsweise vor dem Vermischen mit Wasser, vorzugsweise in einem Verhältnis von etwa 10 : 1 bis 1 : 10, vermischt.

Der pH-Wert des gebrauchsfertigen erfindungsgemäßen Färbemittels liegt im sauren bis alkalischen Bereich und beträgt 3,5 bis 12,0, wobei ein pH-Wert von 4,5 bis 10 besonders bevorzugt ist:

Das so erhaltene gebrauchsfertige Mittel zum Färben von Keratinfasern, insbesondere menschlichen Haaren, ('Färbebrei') wird auf das trockene oder handtuchfeuchte Haar gleichmäßig aufgetragen und nach einer Einwirkzeit von 5 bis 80 Minuten bei 20 bis 50 °C, vorzugsweise 15 bis 60 Minuten bei Raumtemperatur (20 bis 25 °C ), beziehungsweise 10 bis 50 Minuten unter Wärmeeinwirkung ( 30 bis 50 °C ), mit Wasser ausgespült und getrocknet.

Insbesondere im Falle von emulsionsförmigen, dispersionsförmigen oder supensionsförmigen Zubereitungen ist es jedoch auch möglich, das Färbemittel ohne vorheriges Vermischen mit Wasser direkt auf das mit Wasser angefeuchtete Haar aufzutragen.

Das erfindungsgemäße Färbemittel ermöglicht eine Verstärkung der Färbeleistung gegenüber üblichen Färbemitteln, wobei völlig unerwartet auch im alkalischen Bereich die beschriebene Farbstoffkombination aus Indigo und bestimmten synthetischen direktziehenden Farbstoffen eine Färbung ergibt, die deutlich über den für die verwendeten Farbstoffe zu erwartenden Einzelergebnissen lag. Aufgrund der hohen Intensität der mit der erfindungsgemäßen Farbstoffkombination erzielten Blaufärbungen sind nur geringe Mengen dieser Farbstoffkombination erforderlich. Die erfindungsgemäße Farbstoffkombination ermöglich zudem in Kombination mit anderen färbenden Pflanzenbestandteilen und/oder synthetischen direktziehenden Farbstoffen jede Nuancierung von blaurot bis rotbraun.

Die nachfolgenden Beispiele sollen den Gegenstand näher erläutern, ohne diesen hierauf zu beschränken.

**Beispiele**

**Beispiel 1**: Haarfärbemittel ohne zusätzliche Pflanzenbestandteile

| Nr. | Zusammensetzung | in % | pH-Wert |
|-----|-----------------|------|---------|
| 1.1 | Indigo | 87,0 | 9,2 |
| | HC Blue No. 12 | 1,0 | |
| | Soda | 12,0 | |
| 1.2 | Indigo | 99,0 | 7,4 |
| | HC Blue No. 12 | 1,0 | |

Jeweils 20 Gramm der vorstehenden Färbemittel werden mit 60 g heißem Wasser ( T = 70 Grad Celsius )in einer Mischschale homogen verrührt und auf trockene gebleichte Haare aufgebracht. Nach einer Einwirkungszeit von 30 Minuten bei 50 Grad Celsius werden die Haare mit warmen Wasser ausgespült und getrocknet.

Mit den vorstehenden Färbemitteln wird direkt nach der Färbung ein sattes Dunkelblau erhalten, das sich im Verlauf der Zeit nur noch unwesentlich verändert.

**Beispiel 2**: Haarfärbemittel mit zusätzlichen Pflanzenbestandteilen

| Nr. | Zusammensetzung | Menge [%] | pH-Wert |
|-----|-----------------|-----------|---------|
| 2.1 | Cassia Auriculata | 49,0 | 6,7 |
| | Indigo | 50,0 | |
| | HC Blue No. 12 | 1,0 | |

(fortgesetzt)

| Nr. | Zusammensetzung | Menge [%] | pH-Wert |
|---|---|---|---|
| 2.2 | Cassia Auriculata | 86,0 | 9,2 |
| | Indigo | 1,0 | |
| | HC Blue No. 12 | 1,0 | |
| | Soda | 12,0 | |
| 2.3 | Cassia Auriculata | 37,0 | 9,2 |
| | Indigo | 50,0 | |
| | HC Blue No. 12 | 1,0 | |
| | Soda | 12,0 | |
| 2.4 | Indigo | 87,0 | 9,2 |
| | HC Blue No. 12 | 1,0 | |
| | Soda | 12,0 | |
| 2.5 | Henna Iran | 37,0 | 9,2 |
| | Indigo | 50,0 | |
| | HC Blue No. 12 | 1,0 | |
| | Soda | 12,0 | |

Jeweils 20 Gramm der vorstehenden Färbemittel werden mit 60 g heißem Wasser ( T = 70 Grad Celsius ) in einer Mischschale homogen verrührt und auf trockene gebleichte Haare aufgebracht. Nach einer Einwirkzeit von 30 Minuten bei 50 Grad Celsius werden die Haare mit warmen Wasser ausgespült und getrocknet.

Mit den vorstehenden Färbemitteln werden direkt nach der Färbebehandlung unterschiedlich intensive Blaufärbungen erhalten, die sich im Verlauf der Zeit nur noch unwesentlich verändern.

**Beispiel 3**: Haarfärbemittel ohne zusätzliche Pflanzenbestandteile

| Zusammensetzung | Menge [%] | pH-Wert |
|---|---|---|
| Indigo | 87,0 | 9,5 |
| HC Blue No. 2 | 1,0 | |
| Soda | 12,0 | |

Jeweils 20 Gramm des vorstehenden Färbemittels werden mit 60 g heißem Wasser ( T = 70 Grad Celsius ) in einer Mischschale homogen verrührt und auf trockene gebleichte Haare aufgebracht. Nach einer Einwirkzeit von 30 Minuten bei 50 Grad Celsius werden die Haare mit warmen Wasser ausgespült und getrocknet.

Mit dem vorstehenden Färbemittel wird direkt nach der Färbebehandlung eine intensive blau-rote Färbung erhalten, die sich im Verlauf der Zeit nur noch unwesentlich verändert.

**Beispiel 4:** Haarfärbemittel mit zusätzlichen Pflanzenbestandteilen

| Zusammensetzung | Menge [%] | pH-Wert |
|---|---|---|
| Cassia Auriculata | 37,0 | 9,4 |
| Indigo | 50,0 | |
| Hc Blue No. 2 | 1,0 | |
| Soda | 12,0 | |

Jeweils 20 Gramm des vorstehenden Färbemittels werden mit 60 g heißem Wasser ( T = 70 Grad Celsius ) in einer Mischschale homogen verrührt und auf trockene gebleichte Haare aufgebracht. Nach einer Einwirkzeit von 30 Minuten bei 50 Grad Celsius werden die Haare mit warmen Wasser ausgespült und getrocknet.

Mit dem vorstehenden Färbemittel wird direkt nach der Färbebehandlung eine intensive blau-rote Färbung erhalten, die sich im Verlauf der Zeit nur noch unwesentlich verändert.

**Beispiel 5:** Haarfärbemittel in Form einer Mehrkomponenten-Zubereitung

Komponente 1: Färbemittelemulsion

| | |
|---|---|
| 1,5 g | Cetylstearylalkohol |
| 2,5 g | Cetyltrimethylammoniumchlorid |
| 2,0 g | HC Red No. 13 |
| 94,0 g | Wasser |
| 100,00 g | |

Der pH-Wert der Färbemittelemulsion wird mit Natriumhydroxid auf pH= 10 eingestellt.

Komponente 2: Pflanzenputver

| | |
|---|---|
| 50,0 g | Indigo |
| 50,0 g | Cassia Auriculata |
| 100,00 g | |

20 g der vorstehend beschriebenen Komponente 1 und 20 Gramm der Komponente 2 werden mit 40 g heißem Wasser in einer Schale mit einem Pinsel oder einem Schneebesen zu einem homogenen "Färbebrei" verrührt. Der pH-Wert des erhaltenen gebrauchsfertigen Färbemittels beträgt 6,4. Das Färbemittel wird sodann mit einem Pinsel auf dunkelblonde Haare (50 % Grauanteil) gleichmäßig aufgetragen. Anschließend wird das Haar 40 Minuten lang mit einer Trockenhaube oder einem Wärmestrahler auf 40 °C erwärmt. Sodann werden die Haare mit warmem Wasser gründlich ausgespült und getrocknet.

Es wird eine intensive blaurote Färbung erhalten, deren Farbton auch nach mehreren Tagen nahezu unverändert ist.

**Beispiel 6:** Haarfärbemittel in Form einer Mehrkomponenten-Zubereitung

Komponente 1: Färbemittelemulsion

| | |
|---|---|
| 1,5 g | Cetylstearylalkohol |

(fortgesetzt)

| | |
|---|---|
| 2,5 g | Cetyltrimethylammoniumchlorid |
| 2,0 g | HC Blue No. 12 |
| 94,0 g | Wasser |
| 100,00 g | |

Der pH-Wert der Färbemittelemulsion wird mit Natriumhydroxid auf pH= 10 eingestellt.

Komponente 2: Pflanzenpulver

| | |
|---|---|
| 92,0 g | Indigo |
| 8,0 g | Natriumcarbonat |
| 100,00 g | |

20 g der vorstehend beschriebenen Komponente 1 und 20 Gramm der Komponente werden mit 40 g heißem Wasser in einer Schale mit einem Pinsel oder einem Schneebesen zu einem homogenen "Färbebrei" verrührt. Der pH-Wert des erhaltenen Färbemittels beträgt 8,9. Das Färbemittel wird sodann mit einem Pinsel auf hellbraune Haare gleichmäßig aufgetragen. Anschließend wird das Haar 30 Minuten lang mit einer Trockenhaube oder einem Wärmestrahler auf 40 °C erwärmt. Sodann werden die Haare mit warmem Wasser gründlich ausgespült und getrocknet.
Es wird eine intensive blau-violette Färbung erhalten, deren Farbton auch nach mehreren Tagen nahezu unverändert ist.

**Beispiel 7:** Cremeförmige Färbemittelsuspension

| | |
|---|---|
| 45,0 g | Isopropylpalmitat |
| 38,5 g | Indigo |
| 10,0 g | Natriumcarbonat |
| 3,0 g | Polyethylenglykol(35)rizinusöl (Cremophor EL der Firma BASF) |
| 1,5 g | Guar Gum |
| 1,4 g | HC Red No. 13 |
| 0,6 g | HC Blue No. 12 |
| 100,00 g | |

30 g der vorstehend beschriebenen Färbemittelsuspension werden mit 60 g heißem (T = 70 °C) Wasser in einer Schale mit einem Pinsel oder einem Schneebesen zu einem homogenen Färbebrei verrührt. Der so erhaltene Färbebrei wird mit einem Pinsel auf dunkelblonde Haare (50 % Grauanteil) gleichmäßig aufgetragen. Anschließend wird das Haar mit einer Folie abgedeckt und 40 Minuten lang mit einer Trockenhaube oder einem Wärmestrahler auf 40 °C erwärmt. Sodann werden die Haare mit warmem Wasser gründlich ausgespült und getrocknet.
Es wird eine intensive blaurote Färbung erhalten.
Alle Prozentangaben betreffen, sofern nicht anders angegeben, Gewichtsprozente.

**Patentansprüche**

1. Mittel zum Färben von Keratinfasern, insbesondere menschlichen Haaren, **dadurch gekennzeichnet, daß** es eine Kombination aus (a) getrockneten und gemahlenen Indigoblättem und (b) mindestens einem synthetischen direktziehenden Farbstoff ausgewählt aus der Gruppe bestehend aus 4-[Ethyl-(2-hydroxyethyl)amino]-1-[(2-hydroxyethyl)amino]-2-nitrobenzol, 1-(2,3-Dihydroxypropyl)amino-2-nitro-4-[methyl-(2-hydroxyethyl)amino]-benzol, 1-(2,3-Dihydroxy-propyl)amino-2-nitro-4-[ethyl-(2-hydroxyethyl)amino]-benzol, 1-(3-Hydroxypropyl)amino-2-nitro-4-bis-(2-hydroxyethylamino)-benzol, 1-(2-Hydroxyethyl)amino-2-nitro-4-aminobenzol und 1-Amino-4-[di(2-hydroxyethyl)amino]-2-nitrobenzol oder deren Salzen, enthält.

**2.** Mittel nach Anspruch 1, **dadurch gekennzeichnet, daß** der synthetische direktziehende Farbstoff (b) ausgewählt ist aus der Gruppe bestehend aus 4-[Ethyl-(2-hydroxyethyl)amino]-1-[(2-hydroxyethyl)-amino]-2-nitrobenzol und 1-Amino-4-[di(2-hydroxyethyl)-amino]-2-nitrobenzol oder deren Salzen.

**3.** Mittel nach Anspruch 1 oder 2, **dadurch gekennzeichnet, daß** es die getrockneten und gemahlenen Indigoblätter in einer Menge von 0,5 bis 99,95 Gewichtsprozent enthält.

**4.** Mittel nach einem der Ansprüche 1 bis 3, **dadurch gekennzeichnet, daß** es die synthetischen direktziehenden Farbstoffe in einer Menge von 0,05 bis 10 Gewichtsprozent enthält.

**5.** Mittel nach einem der Ansprüche 1 bis 4, **dadurch gekennzeichnet, daß** es zusätzlich mindestens einen üblichen, physiologisch unbedenklichen, direktziehenden Farbstoff aus der Gruppe der Nitrofarbstoffe, Azofarbstoffe, Chinonfarbstoffe und Triphenylmethanfarbstoffe enthält.

**6.** Mittel nach einem der Ansprüche 1 bis 5, **dadurch gekennzeichnet, daß** es zusätzlich weitere färbende oder nicht-färbende pflanzliche Bestandteile enthält.

**7.** Mittel nach einem der Ansprüche 1 bis 6, **dadurch gekennzeichnet, daß** es in Form einer Einkomponenten-Zubereitung vorliegt.

**8.** Mittel nach einem der Ansprüche 1 bis 6, **dadurch gekennzeichnet, daß** es in Form einer Mehrkomponenten-Zubereitung vorliegt, wobei die natürlichen Bestandteile getrennt von den übrigen Bestandteilen abgepackt werden.

**9.** Mittel nach Anspruch 8, **dadurch gekennzeichnet, daß** die natürlichen Bestandteile mit den übrigen Bestandteilen des Färbemittels in einem Verhältnis von 10 : 1 bis 1 : 10 vermischt werden.

**10.** Mittel nach einem der Ansprüche 1 bis 9, **dadurch gekennzeichnet, daß** es vor der Anwendung mit Wasser in einem Verhältnis von 1 : 1 bis 1 : 100 zu dem gebrauchsfertigen Färbemittel vermischt wird.

**11.** Mittel nach einem der Ansprüche 1 bis 10, **dadurch, gekennzeichnet, daß** das gebrauchsfertige Färbemittel einen pH-Wert von 3,5 bis 12 aufweist.

**Claims**

**1.** Agent for the colouring of keratin fibres, in particular human hair, **characterized in that** it comprises a combination of (a) dried and ground indigo leaves and (b) at least one synthetic direct dye chosen from the group consisting of 4-[ethyl-(2-hydroxyethyl)amino]-1-[(2-hydroxyethyl)amino]-2-nitrobenzene, 1-(2,3-dihydroxypropyl)amino-2-nitro-4-[methyl-(2-hydroxyethyl)-amino]benzene, 1-(2,3-dihydroxypropyl)amino-2-nitro-4-[ethyl-(2-hydroxyethyl)amino]benzene, 1-(3-hydroxypropyl)amino-2-nitro-4-bis(2-hydroxyethylamino]benzene, 1-(2-hydroxyethyl)amino-2-nitro-4-aminobenzene and 1-amino-4-[di(2-hydroxyethyl)amino]-2-nitrobenzene or salts thereof.

**2.** Agent according to Claim 1, **characterized in that** the synthetic direct dye (b) is chosen from the group consisting of 4-[ethyl-(2-hydroxyethyl)amino]-1-[(2-hydroxyethyl)amino]-2-nitrobenzene and 1-amino-4-[di(2-hydroxyethyl)amino]-2-nitrobenzene or salts thereof.

**3.** Agent according to Claim 1 or 2, **characterized in that** it comprises the dried and ground indigo leaves in an amount of from 0.5 to 99.95 percent by weight.

**4.** Agent according to one of Claims 1 to 3, **characterized in that** it comprises the synthetic direct dyes in an amount of from 0.05 to 10 percent by weight.

**5.** Agent according to one of Claims 1 to 4, **characterized in that** it additionally comprises at least one customary, physiologically acceptable, direct dye from the group of nitro dyes, azo dyes, quinone dyes and triphenylmethane dyes.

**6.** Agent according to one of Claims 1 to 5, **characterized in that** it additionally comprises further colouring or non-colouring plant constituents.

**7.** Agent according to one of Claims 1 to 6, **characterized in that** it is in the form of a single-component preparation.

**8.** Agent according to one of Claims 1 to 6, **characterized in that** it is in the form of a multicomponent preparation, where the natural constituents are packaged separately from the other constituents.

**9.** Agent according to Claim 8, **characterized in that** the natural constituents are mixed with the other constituents of the colorant in a ratio of from 10:1 to 1:10.

**10.** Agent according to one of Claims 1 to 9, **characterized in that** it is mixed prior to the application with water in a ratio of from 1:1 to 1:100 to give the ready-to-use colorant.

**11.** Agent according to one of Claims 1 to 10, **characterized in that** the ready-to-use colorant has a pH of from 3.5 to 12.


**Revendications**

**1.** Composition pour la teinture de fibres de kératine, en particulier de cheveux humains, **caractérisée en ce qu'**elle contient une association de (a) feuilles d'indigotier broyées et séchées et (b) au moins un colorant direct de synthèse choisi dans le groupe constitué par le 4-[éthyl-(2-hydroxyéthyl)amino]-1-[(2-hydroxyéthyl)-amino]-2-nitrobenzène, le 1-(2,3-dihydroxypropyl)-amino-2-nitro-4-[méthyl-(2-hydroxyéthyl)amino]-benzène, le 1-(2,3-dihydroxypropyl)amino-2-nitro-4-[éthyl-(2-hydroxyéthyl)amino]-benzène, le 1-(3-hydroxypropyl)amino-2-nitro-4-bis(2-hydroxyéthylamino]-benzène, le 1-(2-hydroxyéthyl)amino-2-nitro-4-aminobenzène et le 1-amino-4-[di(2-hydroxyéthyl)amino]-2-nitrobenzène ou leurs sels.

**2.** Composition selon la revendication 1, **caractérisée en ce que** le colorant direct (b) de synthèse est choisi dans le groupe constitué par le 4-[éthyl-(2-hydroxyéthyl)amino]-1-[(2-hydroxyéthyl)amino]-2-nitrobenzène et le 1-amino-4-[di(2-hydroxyéthyl)amino]-2-nitrobenzène ou leurs sels.

**3.** Composition selon la revendication 1 ou 2, **caractérisée en ce qu'**elle contient les feuilles d'indigotier broyées et séchées en une quantité de 0,5 à 99,95 % en poids.

**4.** Composition selon l'une quelconque des revendications 1 à 3, **caractérisée en ce qu'**elle contient les colorants directs de synthèse en une quantité de 0,05 à 10 % en poids.

**5.** Composition selon l'une quelconque des revendications 1 à 4, **caractérisée en ce qu'**elle contient en outre au moins un colorant direct usuel, physiologiquement acceptable, choisi dans le groupe des colorants nitrés, des colorants azoïques, des colorants quinoniques et des colorants triphénylméthane.

**6.** Composition selon l'une quelconque des revendications 1 à 5, **caractérisée en ce qu'**elle contient en outre d'autres composants végétaux colorants ou non colorants.

**7.** Composition selon l'une quelconque des revendications 1 à 6, **caractérisée en ce qu'**elle se trouve sous forme d'une préparation à un seul composant.

**8.** Composition selon l'une quelconque des revendications 1 à 6, **caractérisée en ce qu'**elle se trouve sous forme d'une préparation à plusieurs composants, les composants naturels étant conditionnés séparément des autres composants.

**9.** Composition selon la revendication 8, **caractérisée en ce que** les composants naturels sont mélangés avec les autres composants de la composition de teinture en un rapport de 10:1 à 1:10.

**10.** Composition selon l'une quelconque des revendications 1 à 9, **caractérisée en ce qu'**elle est mélangée avant l'emploi avec de l'eau en un rapport de 1:1 à 1:100 relativement au produit de teinture prêt à l'emploi.

**11.** Composition selon l'une quelconque des revendications 1 à 10, **caractérisée en ce que** le produit prêt à l'emploi présente un pH de 3,5 à 12.